# EUROPÄISCHE PATENTSCHRIFT

(11) **EP 3 579 788 B1**
(45) Veröffentlichungstag und Bekanntmachung des Hinweises auf die Patenterteilung: **13.07.2022**
(21) Anmeldenummer: 18710315.5
(22) Anmeldetag: 09.02.2018
(51) Int. Cl.: A61F 2/24

(54) **BIOLOGISCHE TRANSKATHETERKLAPPE**
BIOLOGICAL TRANSCATHETER VALVE
VALVE TRANSCATHÉTER BIOLOGIQUE

(30) Priorität: 10.02.2017 DE 102017202159
(43) Veröffentlichungstag der Anmeldung: 18.12.2019
(73) Patentinhaber: Tribio GmbH, 74076 Heilbronn (DE)
(72) Erfinder: SIEVERS, Hans-Hinrich, 24119 Kronshagen (DE); SCHARFSCHWERDT, Michael, 23564 Lübeck (DE)
(74) Vertreter: Patentanwälte Vollmann Hemmer Lindfeld Partnerschaft mbB
(86) Internationale Anmeldenummer: PCT/DE2018/200010
(87) Internationale Veröffentlichungsnummer: WO 2018/145705

(56) Entgegenhaltungen:
- US-A1- 2006 122 692
- US-A1- 2009 171 456
- US-A1- 2012 271 398
- US-A1- 2012 310 328
- US-B2- 8 795 354

## Beschreibung

Die Erfindung betrifft eine Herzklappenprothese, welche für eine Transkatheter-Implantation ausgebildet ist, d. h. eine Transkatheter-Herzklappenprothese.

Herzklappenprothesen werden zunehmend über Katheter implantiert. Dieses Verfahren ist als TAVI (transcatheter aortic valve implantation) bekannt. Dabei kann nicht nur die Aortenklappe über einen Katheter implantiert werden, auch die Implantation einer Mitralklappenprothese ist über einen Katheter möglich. Diese Verfahren haben den Vorteil, dass keine Operation am offenen Herzen erforderlich ist. Die Schwierigkeit ist jedoch, dass die Herzklappenprothese für die Implantation sehr klein zusammengefaltet werden muss, um mit Hilfe eines Katheters durch ein Blutgefäß in die gewünschte Position gebracht zu werden. In der gewünschten Position im Herzen wird die Herzklappenprothese dann entfaltet und klemmend an der Gefäßwand fixiert. Da die anatomischen Gegebenheiten von Patient zu Patient unterschiedlich sind, ist es schwierig, die Herzklappenprothese stets optimal zu platzieren. So besteht beispielsweise das Problem, dass der Querschnitt des Blutgefäßes, in welchem die Herzklappenprothese fixiert werden soll, in der Regel nicht ideal kreisförmig ist. So kommt es beim Einsetzen der Herzklappenprothese zu Verformungen der gesamten Herzklappenprothese, welche auch zu Verformungen der Klappensegel führen können. Dies kann zu Funktionsbeeinträchtigungen führen oder die Haltbarkeit der Herzklappenprothese beeinträchtigen. US 2009/171456 A1 zeigt eine perkutane Herzklappe mit einem ausdehnbaren Klappenrahmen mit Klappenrahmenelementen, einem mit dem Klappenrahmen verbundenen Klappensegel und einem mit dem Klappenrahmen verbundenen ausdehnbaren Stent-Verankerungsrahmen mit Stentrahmenelementen.

Im Hinblick auf diese Problematik ist es Aufgabe der Erfindung, eine für eine Transkatheter-Implantation vorgesehene Herzklappenprothese dahingehend zu verbessern, dass die Herzklappenprothese sich besser an die Anatomie des Patienten anpassen kann, ohne zu unerwünschten Funktionsbeeinträchtigungen zu führen.

Diese Aufgabe wird durch für eine Transkatheter-Implantation ausgebildete Herzklappenprothese mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Die erfindungsgemäße Herzklappenprothese ist für die Transkatheter-Implantation ausgebildet, d. h. es handelt sich um eine Transkatheter-Herzklappenprothese, welche derart zusammenfaltbar ist, dass sie über einen Katheter durch ein Blutgefäß, beispielsweise eine Vene oder die Aorta hindurch in das Herz vorgeschoben werden kann. Die erfindungsgemäße Herzklappenprothese kann als Aortenklappenprothese oder auch als Mitralklappenprothese ausgebildet sein.

Die erfindungsgemäße Herzklappenprothese weist eine Tragstruktur auf, an welcher die Klappensegel befestigt sind. Die Tragstruktur besteht im Wesentlichen aus zwei Teilen, nämlich einem zusammenfaltbaren Klappengestell sowie einem Befestigungsring. Dabei liegt der Befestigungsring in der vorgesehenen Durchströmungsrichtung der Herzklappenprothese stromaufwärts des Klappengestells, d. h. als Aortenklappe implantiert der Herzkammer zugewandt. An dem Klappengestell sind mehrere Klappensegel, insbesondere drei Klappensegel, befestigt. Die Anordnung der Klappensegel bildet dabei insbesondere die Anordnung der Klappensegel der natürlichen Aortenklappe nach. Die Klappensegel sind an dem Klappengestell so angeordnet, dass ihre freien Kanten, welche in geschlossenem Zustand der Herzklappenprothese aneinander anliegen und im geöffneten Zustand voneinander beabstandet sind, an dem dem Befestigungsring abgewandten Axialende der Herzklappenprothese gelegen sind. Auch bei Verwendung als Mitralklappenprothese kann eine solche Anordnung von drei Klappensegeln Verwendung finden. Um die drei Klappensegel, welche eine Taschenklappe bilden, zu fixieren, weist das Klappengestell vorzugsweise in bekannter Weise drei bogenförmige Abschnitte auf, wobei die Bögen in drei Kommissuren auslaufen. An den Bögen sind die Klappensegel befestigt. Das Klappengestell hat die Funktion, die Klappensegel zu halten und definiert zueinander zu positionieren.

An das Klappengestell schließt sich in der vorgesehenen Strömungsrichtung durch die Herzklappenprothese stromaufwärts gelegen ein Befestigungsring an, über welchen die Herzklappenprothese im Herzen fixiert wird. Im Bereich des Befestigungsringes wird die Herzklappenprothese somit an der Innenwandung eines Blutgefäßes, d. h. bevorzugt am natürlichen Klappenring zur dichten Anlage gebracht, um die Herzklappenprothese gegenüber dem umgebenden Gewebe abzudichten. So ist der Befestigungsring derart ausgebildet, dass er sich gegen eine umgebende Gefäßwandung des Klappenringes der natürlichen Herzklappe verklemmt. Dazu ist der Befestigungsring ebenfalls zusammenfaltbar ausgebildet, um so zusammengefaltet zu werden, dass er gemeinsam mit dem Klappengestell mit Hilfe eines Katheters in das Herz vorgeschoben werden kann. In der gewünschten Position wird der Befestigungsring dann so erweitert, dass er gegen die Gefäßwandung gedrückt wird und dort kraft- und/oder formschlüssig fixiert wird und so die Herzklappenprothese in einer gewünschten Position und dicht im Herzen fixiert. Abhängig von der jeweiligen Anatomie des Herzen kann es dabei zu einer Verformung des Befestigungsringes kommen. In seiner entfalteten Grundform hat der Befestigungsring vorzugsweise einen im Wesentlichen kreisförmigen Querschnitt.

Die Herzklappenprothese ist so ausgebildet, dass auch im implantierten Funktionszustand der Befestigungsring an einem ersten Axialende und das Klappengestell mit den Klappensegeln axial an diesem anschließend gelegen ist, wobei der Befestigungsring in der vorgesehenen Durchflussrichtung stromaufwärts gelegen ist. Die Verformbarkeit bzw. Kraftentkopplung ist somit erfindungsgemäß auch im implantierten Funktionszustand gegeben. Das Klappengestell mit den Klappensegeln weist gegenüber dem Befestigungsring, an welchem die Herzklappenprothese im Blutgefäß befestigt und an diesem abgedichtet ist, eine gewisse Beweglichkeit bzw. Verformbarkeit auf, welche sicherstellt, dass das Klappengestell nach der Implantation seine gewünschte definierte Form unabhängig von der Befestigung des Befestigungsringes einnehmen kann.

Erfindungsgemäß ist vorgesehen, dass der Befestigungsring mit dem Klappengestell derart kraftentkoppelt verbunden ist, dass eine gewisse Verformung des Befestigungsringes möglich ist, ohne dass es zu einer wesentlichen Verformung des Klappengestells kommt. D. h., eine Verformung des Befestigungsringes wird zumindest nur eingeschränkt und bevorzugt überhaupt nicht auf das Klappengestell übertragen. Dies ermöglicht es, dass es bei der Befestigung des Befestigungsringes im Blutgefäß, insbesondere im Klappenring einer natürlichen Herzklappe, zu einer gewissen Verformung des Befestigungsringes kommen kann, ohne dass das Klappengestell wesentlich verformt wird. D. h., das Klappengestell behält seine im entfalteten Zustand vorgesehene definierte Form bei, so dass auch die Klappensegel nicht verformt werden und in der gewünschten Weise zueinander positioniert sind. Diese Kraftentkopplung zwischen Befestigungsring und Klappengestell stellt somit die vorgesehene Funktionsfähigkeit der Herzklappenprothese sicher, auch wenn es zu einer Verformung des Befestigungsbereiches bzw. Befestigungsringes der Herzklappenprothese, mit welchem die Herzklappenprothese im Blutgefäß fixiert wird, kommen sollte. Die Herzklappenprothese ist somit so ausgestaltet, dass derjenige Bereich, welcher zur Befestigung und Abdichtung dient, nämlich der Befestigungsring, gegenüber demjenigen Teil, welcher die Klappensegel trägt, d. h. dem Klappengestell, kraftentkoppelt ist. So wird insgesamt die Zuverlässigkeit und Haltbarkeit der Herzklappenprothese erhöht.

Vorzugsweise sind das Klappengestell und/oder der Befestigungsring selbstentfaltend ausgebildet. D. h., beim Zusammenfalten auf den verkleinerten Durchmesser zum Anordnen in einem Katheter wird das Material des Klappengestells und/oder das Material des Befestigungsringes elastisch verformt. In diesem verformten Zustand werden das Klappengestell und/oder der Befestigungsring dann durch geeignete Haltemittel in ihrem verkleinerten Durchmesser gehalten. Wenn die Haltemittel nach Erreichen der gewünschten Position gelöst werden, können sich das Klappengestell und/oder der Befestigungsring dann selbsttätig wieder in ihre Ausgangsform zurück verformen. Der Befestigungsring ist dabei vorzugsweise so ausgebildet, dass sein Außendurchmesser geringfügig größer ist als der Innendurchmesser des natürlichen Klappenringes, in welchen die Herzklappenprothese eingebracht werden soll. Dadurch wird erreicht, dass der Befestigungsring auch nach dem Entfalten um ein gewisses Maß elastisch verformt bleibt, sodass durch die elastischen Spannungen in dem Befestigungsring radial nach außen gerichtete Druckkräfte erzeugt werden, welche den Befestigungsring gegen das umgebende Gewebe drücken.

Weiter bevorzugt sind das Klappengestell und/oder der Befestigungsring und/oder gegebenenfalls ein Verbindungsbereich, wie er unten beschrieben wird, zur Entfaltung unter Verwendung zumindest eines Ballons ausgebildet. Zur Entfaltung können dabei gegebenenfalls auch mehrere Ballons verwendet werden. Besonders bevorzugt erfolgt die Entfaltung in der Weise, dass zum Entfalten auf den Befestigungsring und das Klappengestell unterschiedlich große, radial nach außen gerichtete Kräfte von der Innenseite her aufgebracht werden. Dies kann beispielsweise unter Verwendung zweier getrennter Ballons zum Aufweiten des Klappengestells und des Befestigungsringes oder beispielsweise auch unter Verwendung eines Ballons, welcher sich in unterschiedlichen Bereichen unterschiedlich dehnt, bewerkstelligt werden. Da der Befestigungsring bevorzugt eine größere Festigkeit als das Klappengestell aufweist und im Übrigen der Befestigungsring gegen das umgebende Gewebe gedrückt werden muss, ist es bevorzugt, zum Aufweiten in diesem Bereich größere Kräfte aufzubringen.

Weiter bevorzugt sind das Klappengestell und/oder der Befestigungsring aus Metall und bevorzugt aus einem Formgedächtnismaterial gefertigt. Bei dem Formgedächtnismaterial handelt es sich vorzugsweise um ein geeignetes Metall, es können jedoch auch andere Materialien Verwendung finden, welche nach der Umformung bzw. dem Zusammenfalten wieder in ihre vorherige Ausgangsform zurückgebracht werden können. Das Formgedächtnismaterial kann so ausgebildet werden, dass es durch elastische Rückverformungen seine Ausgangsform wieder einnimmt, wie es vorangehend beschrieben wurde. Alternativ können Formgedächtnismaterialien und insbesondere Formgedächtnismetalle Verwendung finden, welche durch Temperaturänderung in ihre Ausgangsform zurückgebracht werden können. Vorzugsweise sind das Klappengestell und/oder der Befestigungsring aus einer Nickel-Titan-Legierung, insbesondere Nitinol, oder einer Kobalt-Chrom-Legierung gefertigt. Der Befestigungsring und das Klappengestell sind bevorzugt aus demselben Material gefertigt, können jedoch alternativ auch aus unterschiedlichen Materialien gefertigt sein.

Das Klappengestell und der Befestigungsring sind vorzugsweise über einen verformbaren Verbindungsbereich miteinander verbunden. Der Verbindungsbereich weist bevorzugt ebenfalls eine tragende Struktur auf und ist aus zwei Gründen verformbar. Zum einen weist der Verbindungsbereich eine Verformbarkeit auf, welche ein Zusammenfalten ermöglicht, um den Verbindungsbereich zusammen mit dem Klappengestell und dem Befestigungsring zum Einbringen in einen Katheter zusammenzufalten. Zum anderen ist der Verbindungsbereich jedoch so ausgebildet, dass er auch in seinem entfalteten Zustand eine Verformbarkeit aufweist, insbesondere eine leichtere Verformbarkeit als das Klappengestell. Es kann sich dabei um eine elastische und/oder plastische Verformbarkeit handeln. Die Verformbarkeit des Verbindungsbereiches ermöglicht es diesem, Verformungen, welche durch eine Verformung des Befestigungsringes beim Einsetzen in das Herz hervorgerufen sind, so aufzunehmen, dass diese Verformungen nicht auf das Klappengestell übertragen werden. D. h., durch Verformungen des Befestigungsringes hervorgerufene Kräfte werden im Verbindungsbereich absorbiert und so nicht oder im Wesentlichen nicht auf das Klappengestell übertragen, so dass das Klappengestell im entfalteten Zustand in einer definierten vorgegebenen Form verbleibt. So können die am Klappengestell befestigten Klappensegel ihre vorgegebene Funktion ohne Beeinträchtigung ausführen.

Bevorzugt ist der Verbindungsbereich aus demselben Material wie das Klappengestell und/oder wie der Befestigungsring gefertigt. Besonders bevorzugt sind sowohl der Verbindungsbereich, das Klappengestell und der Befestigungsring aus einem Metall gefertigt, wie es vorangehend beschrieben wurde, besonders bevorzugt einer Nickel-Titan-Legierung, z. B. Nitinol, oder einer Kobalt-Chrom-Legierung. Das Klappengestell, der Verbindungsbereich und der Befestigungsring sind weiter bevorzugt einstückig ausgebildet.

Gemäß einer alternativen Ausführungsform können der Befestigungsring und das Klappengestell und gegebenenfalls auch der Verbindungsbereich als getrennte Bauteile ausgebildet sein, welche in geeigneter Weise miteinander verbunden sind. Eine solche Ausgestaltung bietet sich insbesondere an, wenn die genannten Teile aus unterschiedlichen Materialien ausgebildet sind. Eine Verbindung kann dabei z. B. formschlüssig, kraftschlüssig und/oder stoffschlüssig erfolgen, beispielsweise durch Verschweißen. Auch ist eine Verbindung gegebenenfalls über ein weiteres Element, beispielsweise ein umhüllendes Gewebematerial, wie es unten beschrieben wird, möglich.

Besonders bevorzugt sind das Klappengestell, der Verbindungsbereich und der Befestigungsring bzw. deren tragende Struktur aus einem metallischen Rohr geschnitten. Ggf. kann das Rohr vor und/oder nach dem Schneidevorgang umgeformt werden, um dem Klappengestell, dem Befestigungsring und dem Verbindungsbereich die gewünschte endgültige geometrische Form zu geben, welche diese nach dem Entfalten im Herzen einnehmen sollen. Ggf. kann in diesem Prozess ein Wärmebehandlungsschritt bzw. ein Härten vorgesehen sein, um beispielsweise einem Formgedächtnismaterial wie Nitinol die gewünschte Sollform "einzubacken".

Der Verbindungsbereich weist vorzugsweise eine Struktur auf, welche eine geringere Festigkeit als das Klappengestell aufweist. So wird eine leichtere Verformbarkeit des Verbindungsbereiches als des Klappengestells erreicht, so dass der Verbindungsbereich bei Verformung des Befestigungsringes die dadurch resultierenden Kräfte und Spannungen durch eine plastische und/oder elastische Verformung der Struktur des Verbindungsbereiches aufnehmen bzw. kompensieren kann. Somit werden vom Befestigungsring bzw. dessen Verformung hervorgerufene Kräfte und Spannungen nicht auf das Klappengestell übertragen.

So kann der Verbindungsbereich weiter bevorzugt eine Struktur aufweisen, welche eine geringere Materialstärke und/oder eine dünnere Ausbildung und/oder eine andere Form ihrer tragenden Elemente aufweist als eine Struktur des Befestigungsringes und/oder des Klappengestells. Eine andere Form der tragenden Struktur kann beispielsweise durch unterschiedlich große Ausnehmungen in den verschiedenen Teilen oder aber beispielsweise unterschiedlich große Maschen einer Gitterstruktur, wie sie unten beschrieben wird, erreicht werden. D. h., insbesondere bei einer einstückigen Ausgestaltung des Verbindungsbereiches mit dem Klappengestell und dem Befestigungsring kann der Verbindungsbereich aus demselben Material ausgebildet sein und die unterschiedliche Verformbarkeit wird allein durch die geometrische Gestaltung der Struktur des Verbindungsbereiches erreicht. So ist das Material im Verbindungsbereich bevorzugt ausgedünnt, so dass es sich in diesem Bereich leichter verformen kann.

Der Verbindungsbereich und das Klappengestell und weiter bevorzugt auch der Befestigungsring sind bevorzugt als eine zusammenfaltbare Gitterstruktur ausgebildet. Die Gitterstruktur ist so ausgebildet, dass Maschen bzw. Waben gebildet werden, welche beim radialen Zusammendrücken der Herzklappenprothese verkleinert werden, wobei die Struktur gleichzeitig in der axialen Länge gestreckt wird. D. h., die Gitterstruktur ähnelt der Struktur eines Streckmetalls, wobei beim Verkleinern des Durchmessers der Herzklappenprothese die Gitterstege zusammengeschoben werden, so dass sie vorzugsweise direkt zur Anlage kommen und die Freiräume zwischen den Gitterstegen verkleinert bzw. eliminiert werden. Die Maschen weisen bevorzugt eine rautenähnliche Gestalt auf, so dass die Gitterstege durch Biegung bzw. Verformung in den Eckpunkten in parallele Ausrichtung zueinander gebracht werden können, in welcher der Durchmesser der Herzklappenprothese minimiert ist, um in einen Katheter eingesetzt zu werden. Bevorzugt ist die gesamte Herzklappenprothese, bestehend aus Klappengestell, Verbindungsbereich und Befestigungsring als eine solche Gitterstruktur ausgebildet. Die Schwächung bzw. leichtere Verformbarkeit des Verbindungsbereiches kann dabei bevorzugt durch dünnere Gitterstege und/oder auch und/oder größere Maschen bzw. Freiräume im Bereich des Verbindungsbereiches im Vergleich mit dem Klappengestell und/oder dem Befestigungsring erreicht werden. So ist vorzugsweise zumindest ein Teil der Gitterstege des Verbindungsbereiches schmaler ausgebildet als die Gitterstege des Klappengestells und vorzugsweise auch als die Gitterstege des Befestigungsringes.

Wie oben bereits beschrieben, weist das Klappengestell mehrere Kommissuren, insbesondere drei Kommissuren mit zwischen diesen gelegenen Bögen auf, an welchen die Klappensegel befestigt sind, wobei im entfalteten Zustand der Herzklappenprothese die Scheitelbereiche der Bögen radial gegenüber dem Befestigungsring auskragen. D. h., im Mittelbereich jedes Bogens, welcher den Scheitelbereich definiert, bildet der Bogen eine radial nach außen gerichtete Ausstülpung gegenüber dem Befestigungsring und dem freien Ende des Klappengestells, d. h., der von den Spitzen der Kommissuren aufgespannten Umfangslinie. Diese Ausstülpung hat den Vorteil, dass sich im implantierten Zustand diese Bereiche der Herzklappenprothese in die Sinus aortae hinein erstrecken können. Dadurch wird die Befestigung der Klappensegel in diesem Bereich aus dem freien Strömungsquerschnitt nach außen verlagert, so dass insgesamt ein größerer freier Strömungsquerschnitt erreicht werden kann. Die radial auskragende Gestalt ist dem Klappengestell bei Ausbildung bzw. Formgebung seiner Struktur vor dem Zusammenfalten gegeben worden, wobei das Material des Klappengestells so ausgebildet ist, dass nach der Implantation und dem Auseinanderfalten die Auskragungen ihre zuvor definierte Form wieder einnehmen.

Eine Ausgestaltung des Klappengestells in der vorangehend beschriebenen Weise, bei welcher sich die Bögen des Klappengestells im entfalteten Zustand in die Sinus aortae hinein erstrecken können, eignet sich insbesondere, wenn die Herzklappenprothese in die natürliche Herzklappe zu deren Ersatz eingesetzt wird. Eine solche Ausgestaltung kann jedoch auch verwendet werden, wenn die erfindungsgemäße Herzklappenprothese in eine zuvor konventionell implantierte biologische Herzklappenprothese als Ersatz eingesetzt werden soll. Dies gilt insbesondere dann, wenn die zu ersetzende biologische Herzklappenprothese eine ähnliche Form aufweist, bei welcher sich Teile des Klappengestells bereits in die Sinus aortae hinein erstrecken. Alternativ kann die erfindungsgemäße Herzklappenprothese so ausgebildet sein, dass sich das Klappengestell in radialer Richtung nicht über den Befestigungsring oder im Wesentlichen nicht über den Befestigungsring hinaus nach außen erstreckt. Eine solche Ausgestaltung der Herzklappenprothese eignet sich, wenn die Herzklappenprothese dazu genutzt werden soll, eine zuvor über einen Katheter implantierte Herzklappenprothese, welche keine Aufweitungen in die Sinus aortae hinein aufweist, zu ersetzen. Wenn die zu ersetzende Herzklappenprothese keine Freiräume aufweist, in welche sich Teile des Klappengestells hinein erstrecken können, ist es bevorzugt, die erfindungsgemäße Herzklappenprothese in einer im Wesentlichen zylindrischen Außengestalt auszubilden, um so die Implantation in eine konventionelle TAVI-Herzklappenprothese hinein zu ermöglichen.

Abweichend oder zusätzlich zu den vorangehend beschriebenen Ausstülpungen ist es erfindungsgemäß möglich, die tragende Struktur der Herzklappenprothese so auszugestalten, dass es einzelne, in radialer Richtung nach innen oder nach außen gerichtete Ausstülpungen gibt, welche sich jeweils nur über einen begrenzten Umfangsbereich der Struktur erstrecken. Dies können insbesondere die vorangehend beschriebenen Ausstülpungen des Klappengestells sein.

Gemäß einer weiteren bevorzugten Ausführungsform der Erfindung definiert das Klappengestell mehrere Kommissuren, insbesondere drei Kommissuren, welche an ihren freien Enden über einen faltbaren Verbindungsring miteinander verbunden sind. Die Faltbarkeit des Verbindungsringes ermöglicht es, dass dieser mit der gesamten Herzklappenprothese zum Einbringen in einen Katheter zusammengefaltet werden kann. Im entfalteten Zustand hält der Verbindungsring die freien Enden der Kommissuren zusammen und gibt diesen zusätzliche Stabilität.

Das Klappengestell und der Befestigungsring sowie der diese verbindende Verbindungsbereich sind ferner zweckmäßigerweise umhüllt und insbesondere mit einem Gewebematerial umhüllt. Dieses Gewebematerial kann wie bei Herzklappenprothesen üblich ausgebildet sein. Es verschließt bzw. überdeckt insbesondere die Freiräume einer Gitterstruktur des Klappengestells und des Verbindungsbereiches und stellt somit die Dichtigkeit der Herzklappenprothese nach außen her. Die Umhüllung des Klappengestells und des Befestigungsringes sichert die Abdichtung der Herzklappenprothese nach außen, so dass lediglich ein definierter Strömungsweg durch das Innere und das von den Klappensegeln begrenzte Lumen hindurch möglich ist. Im Bereich des Befestigungsringes ist die Herzklappenprothese zur Abdichtung gegenüber dem umgebenden Gewebe bei der Implantation vorgesehen bzw. ausgebildet.

Die Klappensegel sind besonders bevorzugt in bekannter Weise aus einem biologischen Material gefertigt.

Gemäß einer weiteren bevorzugten Ausführungsform der erfindungsgemäßen Herzklappenprothese kann diese im Bereich des Befestigungsringes einen oder mehrere ringförmige Befestigungswülste aufweisen, welche den Befestigungsring außenumfänglich umgeben. Diese Befestigungswülste sind vorzugsweise aus elastischem Material, beispielsweise aus Gewebematerial wie Dracon ausgebildet. Die Befestigungswülste dienen dazu, die Herzklappenprothese in dichte Anlage mit dem umgebenden natürlichen Gewebe zu bringen. Dabei kann der Befestigungswulst oder können die Befestigungswülste bevorzugt um ein gewisses Maß verformbar ausgebildet sein, um Unregelmäßigkeiten in der natürlichen Gewebekontur auszugleichen und für eine gute Abdichtung zu sorgen. Eine solche Ausgestaltung ist insbesondere für eine Herzklappenprothese bevorzugt, welche dafür vorgesehen ist, in die natürliche Aortenklappe hinein implantiert zu werden. Für eine Ausgestaltung der Herzklappenprothese, welche zur Implantation in eine früher implantierte Herzklappenprothese vorgesehen ist, wird der Außenumfang des Befestigungsringes bevorzugt glatt und möglichst dünn ausgebildet. In diesem Falle sind, da die Herzklappenprothese an der definierten Innenkontur einer früheren Herzklappenprothese zur Anlage kommen soll, Dichtringe oder Abdichtelemente, welche einen Konturausgleich ermöglichen, nicht erforderlich. Bei der Implantation in eine ältere Herzklappenprothese hinein ist es vielmehr bevorzugt, den Querschnitt so wenig wie möglich einzuschränken, da die zuvor implantierte Herzklappenprothese ebenfalls im Körper verbleibt.

Wie vorangehend bereits angedeutet, eignet sich die erfindungsgemäße Herzklappenprothese neben der Implantation in die natürliche Herzklappe, insbesondere die Aortenklappe, besonders dazu, eine zuvor implantierte Herzklappenprothese zu ersetzen, ohne die zuvor implantierte Herzklappenprothese zu entfernen. D. h., die erfindungsgemäße Herzklappenprothese ist gemäß einer bevorzugten Ausführungsform dazu vorgesehen und so geformt, dass sie in eine ältere Herzklappenprothese eingesetzt werden kann, wobei die zuvor implantierte Herzklappenprothese auseinander gedrückt wird und deren Klappensegel in einen dauerhaft geöffneten Zustand gebracht werden. D. h., die neue Herzklappenprothese wird in das Innere der alten Herzklappenprothese eingesetzt. Diese ältere, zuvor implantierte Herzklappenprothese kann eine konventionell implantierte biologische Herzklappenprothese oder auch eine zuvor implantierte TAVI-Prothese sein. Neben der beschriebenen erfindungsgemäßen Herzklappenprothese ist Gegenstand der Erfindung somit auch ein Implantationsverfahren, bei welchem in eine bestehende Herzklappenprothese die erfindungsgemäße Herzklappenprothese eingesetzt wird. Dabei wird die neue Herzklappenprothese in das Innere der bestehenden Herzklappenprothese im zusammengedrückten Zustand eingeführt und dann expandiert, sodass der Befestigungsring der neuen Herzklappenprothese am Innenumfang der früher implantierten Herzklappenprothese in dichte Anlage kommt und die zuvor implantierte Herzklappenprothese weiter bevorzugt in radialer Richtung aufweitet. Die Klappensegel der älteren Herzklappenprothese werden beim Aufweiten der neuen Herzklappenprothese in eine geöffnete Position auseinander gedrückt. Die Ausgestaltung des Befestigungsringes in einer Weise, dass dieser von dem Klappengestell kraftentkoppelt ist, ermöglicht es, bei diesem Verfahren die alte Herzklappenprothese durch einen relativ starren Befestigungsring aufzuweiten und/oder die neue Herzklappenprothese in feste Anlage am Innenumfang der alten Herzklappenprothese zu bringen. Der Befestigungsring der neuen Herzklappenprothese ermöglicht es somit, relativ große Kräfte in radialer Richtung auf die alte Herzklappenprothese auszuüben. Durch die Kraftentkopplung des Klappengestells wird dabei erreicht, dass das Klappengestell durch die auftretenden Kräfte im Wesentlichen nicht verformt und somit die Funktionalität der Klappensegel im Wesentlichen nicht beeinträchtigt wird. Darüber hinaus kann, wie es oben bevorzugt beschrieben ist, das Klappengestell so ausgebildet sein, dass es sich radial über den Befestigungsring hinaus und besonders bevorzugt in die Sinus aortae hinein erweitern kann.

Nachfolgend wird die Erfindung beispielhaft anhand der beigefügten Figuren beschrieben. In diesen zeigt:
- Fig. 1: schematisch eine erfindungsgemäße Herzklappenprothese im entfalteten Zustand,
- Fig. 2: eine Seitenansicht der tragenden Struktur einer erfindungsgemäßen Herzklappenprothese,
- Fig. 3: eine Schnittansicht der Struktur gemäß Fig. 2 entlang der Linie III-III in Fig. 4,
- Fig. 4: eine Draufsicht auf die Struktur gemäß Fig. 2,
- Fig. 5: eine perspektivische Ansicht der Struktur gemäß Fig. 2,
- Fig. 6: eine perspektivische Ansicht der tragenden Struktur einer erfindungsgemäßen Herzklappenprothese gemäß einer zweiten Ausführungsform der Erfindung,
- Fig. 7: eine Schnittansicht der Struktur gemäß Fig. 6,
- Fig. 8: schematisch eine erfindungsgemäße Herzklappenprothese gemäß einer zweiten Ausführungsform der Erfindung im entfalteten Zustand und
- Fig. 9: schematisch eine erfindungsgemäße Herzklappenprothese gemäß einer dritten Ausführungsform im entfalteten Zustand.

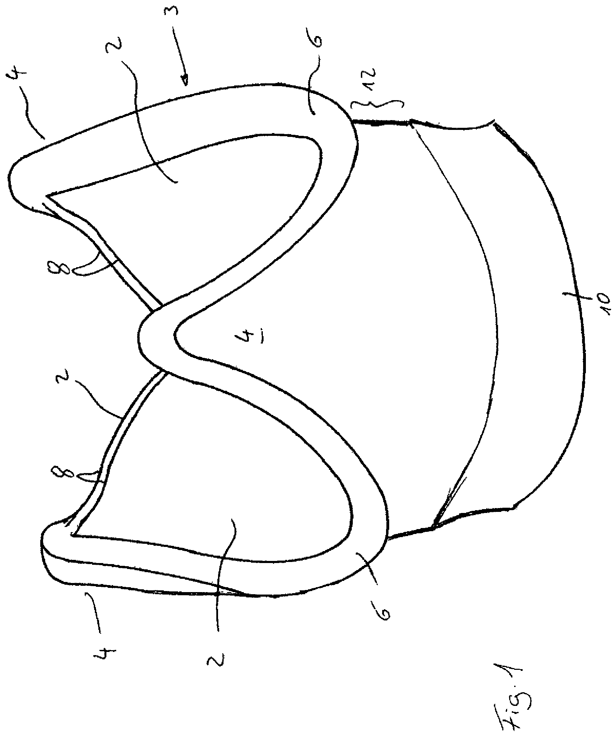

Fig. 1 zeigt schematisch den Gesamtaufbau einer erfindungsgemäßen Herzklappenprothese, welche zum Ersatz der Aortenklappe oder auch der Mitralklappe im Herzen verwendet werden kann. Die Herzklappenprothese ist ausgebildet, um im Rahmen der Transkatheter-Implantation ins Herz eingesetzt zu werden.

Die Herzklappenprothese ist als Taschenklappe mit drei Klappensegeln 2 ausgebildet. Die Klappensegel 2 sind an einem Klappengestell 3 befestigt, welches aus drei Kommissuren 4 gebildet wird, welche durch Verbindungsbögen 6 miteinander verbunden sind. Die Klappensegel 2 sind aus biologischem Material gefertigt und an den Verbindungsbögen 6 befestigt. Die Klappensegel 2 liegen an ihren freien Kanten 8 im geschlossenen Zustand der Herzklappenprothese dichtend aneinander an. Im geöffneten Zustand sind die Kanten 8 voneinander beabstandet.

An dem den Kanten 8 abgewandten Axialende der Herzklappenprothese weist diese einen Befestigungsring 10 auf, mit welchem die Herzklappenprothese in einem Blutgefäß, insbesondere im Bereich des natürlichen Klappenringes, klemmend befestigt wird. Dazu wird der Befestigungsring 10 radial nach außen gedrückt, so dass er an der Wandung des Blutgefäßes zur Anlage kommt. Der Befestigungsring 10 weist eine nach innen gewölbte Gestalt auf, wodurch die axiale Fixierung verbessert wird. Wie in Fig. 1 zu erkennen ist, kragen die Verbindungsbögen 6 radial nach außen über den Befestigungsring 10 aus. So können sich die Verbindungsbögen 6 in die Sinus aortae hinein erstrecken, wodurch der freie Strömungsquerschnitt im Inneren der Herzklappenprothese vergrößert wird.

Der Befestigungsring 10 ist mit dem Klappengestell 3 über einen Verbindungsbereich 12 verbunden, welcher so ausgebildet ist, dass eine Kraftentkopplung zwischen dem Klappengestell 3 und dem Befestigungsring 10 erreicht wird. Dies wird durch eine leichtere Verformbarkeit des Verbindungsbereiches 12 gegenüber dem Klappengestell 3 erreicht. Bei einer Verformung des Befestigungsringes 10 bei der Implantation der Herzklappenprothese wird somit das Klappengestell 3 nicht mit verformt, da der verformbare Verbindungsbereich Formänderungen des Befestigungsringes 10 so ausgleichen kann, dass die Verformungen bzw. Kräfte oder Spannungen im Wesentlichen nicht auf das Klappengestell 3 übertragen werden und die Klappensegel 2 somit in ihrer definierten Lage verbleiben. So wird eine hohe Funktionssicherheit und Haltbarkeit der Herzklappenprothese auch bei einer Verformung des Befestigungsringes 10 erreicht. Eine solche Verformung des Befestigungsringes 10 kann beispielsweise dann auftreten, wenn der Querschnitt des natürlichen Blutgefäßes, in welches der Befestigungsring 10 eingesetzt wird, keine ideale kreisförmige Form hat, was in der Regel der Fall ist.

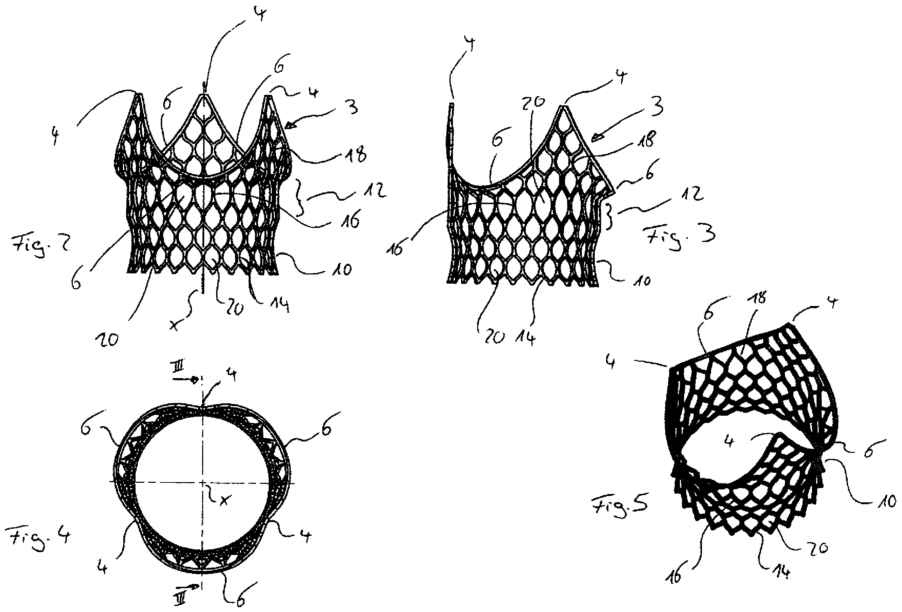

Der Befestigungsring 10, der Verbindungsbereich 12 sowie das Klappengestell 3 werden von einer tragenden Struktur gebildet, welche anhand der Figuren 2 - 7 beschrieben wird. Die tragende Struktur ist von einem Gewebematerial umhüllt, welches in der Darstellung gemäß Figuren 2 - 7 weggelassen worden ist, um die Ausgestaltung der Struktur erkennbar zu machen. Auch die Klappensegel 2 sind in den Figuren 2-7 nicht gezeigt, werden jedoch in der anhand von Fig. 1 beschriebenen Weise angeordnet.

Da die erfindungsgemäße Herzklappenprothese zur Transkatheter-Implantation vorgesehen ist, ist die tragende Struktur zusammenfaltbar ausgebildet. Sie ist, wie in den Fig. 2 - 7 gezeigt, als Gitterstruktur mit rautenförmigen Maschen ausgebildet. Eine solche Gitterstruktur kann, beispielsweise durch Laserschneiden, aus einem rohrförmigen Metallteil ausgeschnitten werden, wobei dieses Metallteil zuvor oder nach dem Schneidvorgang zusätzlich noch umformend bearbeitet werden kann, um die radialen Auskragungen und Einbuchtungen zu schaffen, insbesondere die radial nach innen gerichtete Einbuchtung des Befestigungsringes 10 sowie die radial nach außen gerichtete Auskragung der Verbindungsbögen 6. Durch radial nach innen gerichteten Druck kann die Struktur so zusammengedrückt werden, dass die Gitterstäbe bzw. Gitterstege 14, 16, 18 miteinander in Anlage gebracht werden, d. h., die zwischen ihnen gelegenen Freiräume 20 verkleinert bzw. eliminiert werden. Dabei wird die Struktur gleichzeitig in ihrer axialen Richtung X verlängert. D. h., die Gitterstruktur, welche von den Gitterstegen 14, 16, 18 gebildet wird, hat die Form ähnlich derjenigen eines Streckmetalls, mit dem Unterschied, dass die erweiterte Struktur mit den Freiräumen 20 die Ruhelage darstellt und der Zustand, in welchem die Gitterstege 14, 16, 18 aneinander liegen, den verformten Zustand darstellt.

Die gezeigte Gitterstruktur ist vorzugsweise aus einem Material, insbesondere einem Metall, ausgebildet, welches nach der Verformung wieder zurück in seine Ausgangslage verformt werden kann. Dies kann beispielsweise durch Verwendung eines Formgedächtnismetalls, wie Nitinol, d. h., einer Nickel-Titan-Legierung erreicht werden. Einem solchen Metall kann die Ausgangsstruktur durch Wärmebehandlung "eingebrannt" werden. Beispielsweise durch Temperaturänderung kann das Material in diesen Ausgangszustand zurück verformt werden. Alternativ ist das Material so ausgebildet, dass es beim Zusammendrücken elastisch verformt wird und allein durch die elastischen Spannungen bzw. Federkräfte wieder in seine Ausgangslage zurück verformt wird, wenn die Druckkraft, welche die Struktur in radialer Richtung zusammendrückt, gelöst wird. D. h., beim Lösen eines geeigneten Haltelementes springt die Struktur in ihre Ausgangslage zurück. Dabei erzeugt der Befestigungsring 10 gleichzeitig eine radial nach außen gerichtete Druckkraft, welche für eine feste Anlage im Bereich des Klappenringes der zu ersetzenden Herzklappe sorgt. Neben den beschriebenen Materialien können auch andere geeignete Materialien Verwendung finden, beispielsweise auch eine Kobalt-Chrom-Legierung.

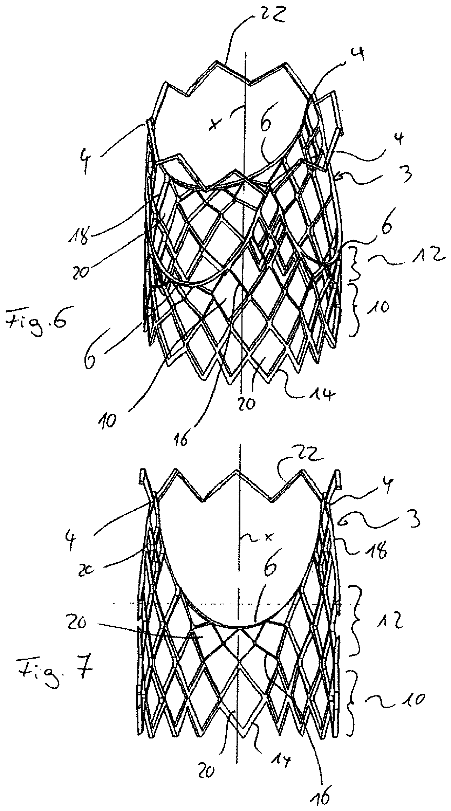

Der Verbindungsbereich 12, welcher den Befestigungsring 10 mit dem Klappengestell 3 verbindet, ist so ausgebildet, dass er leichter verformbar ist als der Befestigungsring 10 bzw. die Gitterstruktur des Befestigungsringes 10 und die Gitterstruktur des Klappengestelles 3. So wird erreicht, dass, wenn es zu einer Verformung des Befestigungsringes 10 aufgrund der anatomischen Verhältnisse kommt, das Klappengestell 3 nicht mit verformt wird. Vielmehr verformt sich der Verbindungsbereich 12 aufgrund seiner leichteren Verformbarkeit so, dass das Klappengestell 3 im Wesentlichen seine vordefinierte, in den Figuren gezeigte Form beibehält. Dies wird dadurch erreicht, dass die Maschen der Gitterstruktur in dem Verbindungsbereich 12, d. h., die Freiräume 20 der Gitterstruktur zwischen den Verbindungsstegen 16 in dem Verbindungsbereich 12 größer ausgebildet sind. D. h., in dem Verbindungsbereich 12 gibt es weniger Material, da dort pro Flächenabschnitt weniger Gitterstege 16 als Gitterstege 14 in einem gleich großen Flächenbereich des Befestigungsringes 10 oder Gitterstege 18 in einem gleich großen Flächenbereich des Klappengestells 3 angeordnet sind. Gleichzeitig können die Gitterstege 16, wie in Fig. 6 und 7 gezeigt, dünner bzw. schmaler ausgebildet sein als die Gitterstege 14 und 18 im Bereich des Befestigungsringes 10 und des Klappengestells 3. Es ist zu verstehen, dass diese schlankere bzw. dünnere Ausgestaltung der Gitterstege 16 auch bei dem Ausführungsbeispiel gemäß Fig. 2 - 5 zur Anwendung kommen könnte.

Das Ausführungsbeispiel gemäß Fig. 6 und 7 unterscheidet sich von dem Ausführungsbeispiel gemäß Fig. 2 - 5 ferner dadurch, dass die Kommissuren 4 an ihren freien Enden zusätzlich durch einen Verbindungsring 22 miteinander verbunden sind. Der Verbindungsring 22 hat einen zickzackförmigen Verlauf, welcher es ermöglicht, auch diesen Verbindungsring 22 zusammenzufalten. Dabei ist der Verbindungsring 22 vorzugsweise aus dem gleichen Material wie auch das Klappengestell 3, der Verbindungsbereich 12 sowie der Befestigungsring 10 ausgebildet, so dass er sich wieder in seine Ausgangslage zurück verformen kann, wenn er in der gewünschten Position im Herzen angeordnet ist. Der Verbindungsring 22 fixiert die Kommissuren 4 zusätzlich und hilft, die definierte Form des Klappengestells 3 und damit die einwandfreie Funktion der Herzklappenprothese zu sichern. Durch den Verbindungsring 22 werden die freien Enden der Kommissuren 4 auseinander gedrückt.

Es ist zu verstehen, dass auch die weiteren Details, welche anhand der Figuren 1 - 5 beschrieben worden sind, bei dem Ausführungsbeispiel gemäß Figuren 6 und 7 realisiert werden könnten. Dies betrifft insbesondere den nach innen gewölbten Befestigungsring 10 sowie die nach außen auskragenden Verbindungsbögen 6, wie sie beispielsweise in Fig. 4 zu erkennen sind.

Besonders bevorzugt ist die gesamte zusammenfaltbare Gitterstruktur, wie sie in den Fig. 2 - 7 gezeigt ist, einstückig aus demselben Material ausgebildet, beispielsweise durch Schneiden aus einem Rohrelement, wie es oben beschrieben wurde.

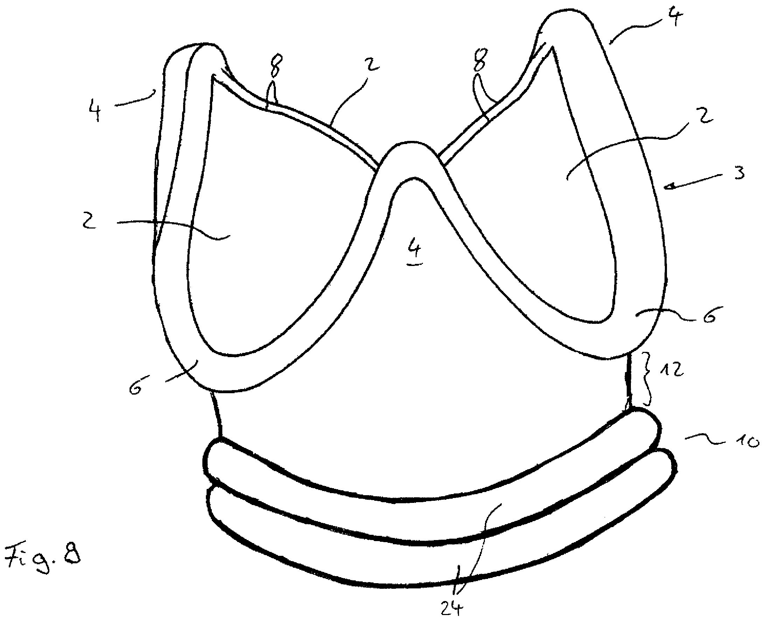

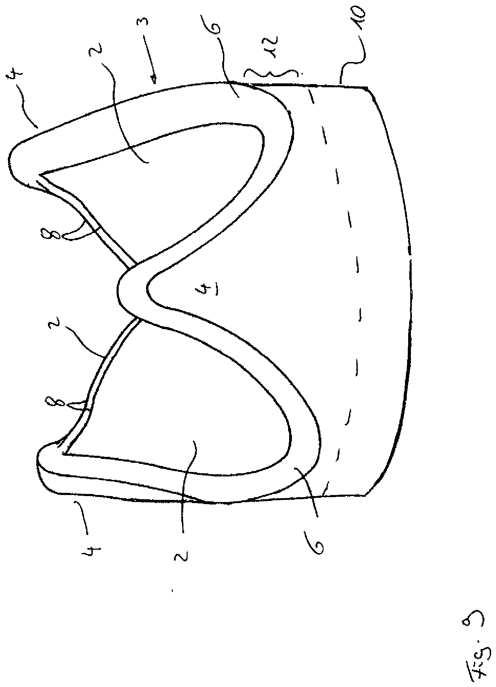

Die Figuren 8 und 9 zeigen zwei alternative Ausführungsformen zu der in Fig. 1 gezeigten Herzklappenprothese, wobei zu verstehen ist, dass der Befestigungsring und das Klappengestell auch bei diesen zwei Ausführungsbeispielen gemäß Fig. 8 und 9 wie anhand der Fig. 2 bis 7 beschrieben ausgebildet sein können. Insbesondere kann zwischen den Kommissuren auch ein Verbindungsring 22 angeordnet sein, wie er vorangehend beschrieben wurde.

Die Ausführungsvariante gemäß Fig. 8 unterscheidet sich von der anhand von Fig. 1 beschriebenen Herzklappenprothese darin, dass im Bereich des Befestigungsringes zwei radial nach außen auskragende Befestigungswülste 24, vorzugsweise aus einem elastischen Gewebematerial wie Dracon, ausgebildet sind. Diese Befestigungswülste 24 werden bei der Implantation gegen das umgebende natürliche Gewebe gedrückt und können durch Verformung Unregelmäßigkeiten im Konturverlauf des Gewebes ausgleichen und so für eine gute Abdichtung der Herzklappenprothese an dem Blutgefäß sorgen.

Das Ausführungsbeispiel gemäß Fig. 9 unterscheidet sich von der Herzklappenprothese, wie sie anhand von Fig. 1 beschrieben wurde, darin, dass die gesamte Herzklappenprothese eine im Wesentlichen zylindrische Außenkontur aufweist, d. h., sich die Verbindungsbögen 6 des Klappengestelles 3 in radialer Richtung im Wesentlichen nicht über den Befestigungsring 10 hinaus erstrecken. Dennoch ist ein Verbindungsbereich 12 zwischen dem Befestigungsring 10 und dem Klappengestell 3 ausgebildet, welcher eine Kraftentkopplung zwischen Klappengestell 6 und Befestigungsring 10 ermöglicht. Dieses Ausführungsbeispiel eignet sich insbesondere zur Implantation in eine zuvor implantierte TAVI-Herzklappenprothese, bei welcher keine Freiräume gegeben sind, in welche sich die Verbindungsbögen 6 gemäß den Ausführungsbeispielen in Fig. 1 und 8 hinein erstrecken könnten. Für das Ausführungsbeispiel gemäß Fig. 9 würde entsprechend auch die tragende Struktur der Herzklappenprothese, wie sie anhand der Fig. 2 bis 6 beschrieben wurde, in einer Weise ausgebildet, dass die Verbindungsbögen 6 sich nicht radial nach außen aufweiten, sondern auch die tragende Struktur eine im Wesentlichen zylindrische Gesamtkontur aufweist.

### Bezugszeichenliste

- 2 -: Klappensegel
- 3 -: Klappengestell
- 4 -: Kommissuren
- 6 -: Verbindungsbögen
- 8 -: Kanten
- 10 -: Befestigungsring
- 12 -: Verbindungsbereich
- 14, 16, 18 -: Gitterstege
- 20 -: Freiräume
- 22 -: Verbindungsring
- 24: Befestigungswülste

- X -: Längsachse

## Patentansprüche

1. Herzklappenprothese, welche für eine Transkatheter-Implantation ausgebildet ist, mit einem zusammenfaltbaren Klappengestell (3), an welchem mehrere Klappensegel (2) befestigt sind, sowie einem sich axial an das Klappengestell (3) anschließenden zusammenfaltbaren Befestigungsring (10), welcher zur Anlage und zur klemmenden Befestigung in einem Blutgefäß ausgebildet ist, **dadurch gekennzeichnet, dass** der Befestigungsring (10) derart kraftentkoppelt mit dem Klappengestell (3) verbunden ist, dass bei der Befestigung in einem Blutgefäß eine Verformung des Befestigungsringes (10) zumindest nur eingeschränkt auf das Klappengestell (3) übertragen wird, und das Klappengestell (3) mehrere Kommissuren (4) mit zwischen diesen gelegenen Verbindungsbögen (6) definiert, an welchen die Klappensegel (2) befestigt sind, wobei im entfalteten Zustand der Herzklappenprothese die Scheitelbereiche der Verbindungsbögen (6) radial gegenüber dem Befestigungsring (10) auskragen, sodass im Mittelbereich jedes Bogens (6), welcher der Scheitelbereich definiert, der Bogen (6) eine radial nach außen gerichtete Ausstülpung gegenüber dem Befestigungsring und einem freien Ende des Klappengestells (3) bildet, welche sich im implantierten Zustand in die Sinus aortae hinein erstrecken können.

2. Herzklappenprothese nach Anspruch 1, **dadurch gekennzeichnet, dass** der Befestigungsring (10) in der vorgesehenen Durchströmungsrichtung der Herzklappenprothese stromaufwärts des Klappengestells (3) gelegen ist.

3. Herzklappenprothese nach Anspruch 1 oder 2, **dadurch gekennzeichnet, dass** das Klappengestell (3) und/oder der Befestigungsring (10) selbstentfaltend oder zur Entfaltung unter Verwendung zumindest eines Ballons ausgebildet sind.

4. Herzklappenprothese nach einem Ansprüche 1 bis 3, **dadurch gekennzeichnet, dass** das Klappengestell (3) und/oder der Befestigungsring (10) aus Metall und bevorzugt aus einer Formgedächtnismaterial gefertigt sind.

5. Herzklappenprothese nach einem der vorangehenden Ansprüche, **dadurch gekennzeichnet, dass** das Klappengestell (3) und/oder der Befestigungsring (10) aus einer Nickel-Titan-Legierung, insbesondere Nitinol, oder einer Kobalt-Chrom-Legierung gefertigt sind.

6. Herzklappenprothese nach einem der vorangehenden Ansprüche, **dadurch gekennzeichnet, dass** das Klappengestell (3) und der Befestigungsring (10) über einen verformbaren Verbindungsbereich (12) mit einander verbunden sind.

7. Herzklappenprothese nach Anspruch 6, **dadurch gekennzeichnet, dass** der Verbindungsbereich (12) aus demselben Material wie das Klappengestell (3) und/oder wie der Befestigungsring (10) gefertigt ist.

8. Herzklappenprothese nach Anspruch 6 oder 7, **dadurch gekennzeichnet, dass** das Klappengestell (3), der Verbindungsbereich (12) und der Befestigungsring (10) einstückig ausgebildet sind.

9. Herzklappenprothese nach einem der Ansprüche 1 bis 7, **dadurch gekennzeichnet, dass** das Klappengestell (3), der Verbindungsbereich (12) und der Befestigungsring (10) mehrteilig ausgebildet sind.

10. Herzklappenprothese nach einem der Ansprüche 6 bis 9, **dadurch gekennzeichnet, dass** der Verbindungsbereich (12) eine Struktur aufweist, welche eine geringere Festigkeit als das Klappengestell (3) aufweist.

11. Herzklappenprothese nach Anspruch 10, **dadurch gekennzeichnet, dass** der Verbindungsbereich (12) eine Struktur aufweist, welche eine geringere Materialstärke und/oder eine dünnere Ausbildung und/oder eine andere Form ihrer tragenden Elemente (16) aufweist als eine Struktur des Klappengestells (3).

12. Herzklappenprothese nach einem der Ansprüche 6 bis 11, **dadurch gekennzeichnet, dass** der Verbindungsbereich (12) und das Klappengestell (3) jeweils als eine zusammenfaltbare Gitterstruktur ausgebildet sind.

13. Herzklappenprothese nach Anspruch 12, **dadurch gekennzeichnet, dass** zumindest ein Teil der Gitterstege (16) des Verbindungsbereiches (12) schmaler ausgebildet ist als die Gitterstege (18) des Klappengestells (3) und/oder zumindest ein Teil der Freiräume (20) zwischen den Gitterstegen (16) in dem Verbindungsbereich (12) größer ausgebildet ist als die Freiräume (20) zwischen den Gitterstegen (18) des Klappengestells (3).

14. Herzklappenprothese nach einem der vorangehenden Ansprüche, **dadurch gekennzeichnet, dass** das Klappengestell (3) mehrere Kommissuren (4) definiert, welche an ihren freien Enden über einen faltbaren Verbindungsring (22) miteinander verbunden sind.

15. Herzklappenprothese nach einem der vorangehenden Ansprüche, **dadurch gekennzeichnet, dass** das Klappengestell (3) und der Befestigungsring (10) sowie der diese verbindende Verbindungsbereich (12) umhüllt sind und insbesondere mit einem Gewebematerial umhüllt sind.

16. Herzklappenprothese nach einem der vorangehenden Ansprüche, **dadurch gekennzeichnet, dass** die Klappensegel (2) aus einem biologischem Material bestehen.

17. Herzklappenprothese nach einem der vorangehenden Ansprüche, **dadurch gekennzeichnet, dass** im Bereich des Befestigungsringes (10) ein oder mehrere ringförmige Befestigungswülste (24) ausgebildet sind.

## Claims

1. A heart valve prosthesis which is designed for a transcatheter implantation, with a collapsible valve frame (3) on which several valve leaflets (2) are fastened, as well as with a collapsible fastening ring (10) which connects axially onto the valve frame (3) and which is designed for the bearing contact and for the clamped fastening in a blood vessel, **characterised in that** the fastening ring (10) is connected to the valve frame (3) in a forcedecoupled manner such that on fastening in a blood vessel, a deformation of the fastening ring (10) is transmitted onto the valve frame (3) at least in an only limited manner, and the valve frame (3) defines several commissures (4) with connection arches (6) which are situated between these and to which the valve leaflets (2) are fastened, wherein in the unfolded state of the heart valve prostheses the apex regions of the connection arches (6) project radially with respect to the fastening ring (10), so that in the middle region of each arch (6) which defines the apex region, the arch (6) forms a radially outwardly directed bulge with respect to the fastening ring and to a free end of the valve frame (3), said bulge being able to extend into the Sinus aortae in the implanted state.

2. A heart valve prosthesis according to claim 1, **characterised in that** the fastening ring (10) is situated upstream of the valve frame (3) in the envisaged flow direction of the heart valve prosthesis.

3. A heart valve prosthesis according to claim 1 or 2, **characterised in that** the valve frame (3) and/or the fastening ring (10) are designed in a self-unfolding manner or for unfolding amid the use of at least one balloon.

4. A heart valve prosthesis according to one of the claims 1 to 3, **characterised in that** the valve frame (3) and/or the fastening ring (10) are manufactured of metal and preferably of a shapememory material.

5. A heart valve prosthesis according to one of the preceding claims, **characterised in that** the valve frame (3) and/or the fastening ring (10) are manufactured from a nickel-titanium alloy, in particular nitinol, or a cobalt-chromium alloy.

6. A heart valve prosthesis according to one of the preceding claims, **characterised in that** the valve frame (3) and the fastening ring (10) are connected to one another via a deformable connection region (12).

7. A heart valve prosthesis according to claim 6, **characterised in that** the connection region (12) is manufactured from the same material as the valve frame (3) and/or as the fastening ring (10).

8. A heart valve prosthesis according to claim 6 or 7, **characterised in that** the valve frame (3), the connection region (12) and the fastening ring (10) are designed as one piece.

9. A heart valve prosthesis according to one of the claims 1 to 7, **characterised in that** the valve frame (3), the connection region (12) and the fastening ring (10) are designed of several parts.

10. A heart valve prosthesis according to one of the claims 6 to 9, **characterised in that** the connection region (12) comprises a structure which has a lower strength than the valve frame (3).

11. A heart valve prosthesis according to claim 10, **characterised in that** the connection region (12) comprises a structure which has a lower material thickness and/or a thinner design and/or a different shape of its supporting elements (16) than a structure of the valve frame (3).

12. A heart valve prosthesis according to one of the claims 6 to 11, **characterised in that** the connection region (12) and the valve frame (3) are each designed as a collapsible lattice structure.

13. A heart valve prosthesis according to claim 12, **characterised in that** at least a part of the lattice webs (16) of the connection region (12) is designed more narrowly than the lattice webs (18) of the valve frame (3) and/or at least a part of the free spaces (20) between the lattice webs (16) in the connection region (12) is designed more largely than the free spaces (20) between the lattice webs (18) of the valve frame (3).

14. A heart valve prosthesis according to one of the preceding claims, **characterised in that** the valve frame (3) defines several commissures (4) which at their free ends are connected to one another via a foldable connection ring (22).

15. A heart valve prosthesis according to one of the preceding claims, **characterised in that** the valve frame (3) and the fastening ring (10) as well as the connection region (12) which connects these are enveloped and in particular are enveloped by a fabric material.

16. A heart valve prosthesis according to one of the preceding claims, **characterised in that** the valve leaflets (2) consist of a biological material.

17. A heart valve prosthesis according to one of the preceding claims, **characterised in that** one or more annular fastening beads (24) are formed in the region of the fastening ring (10).

## Revendications

1. Prothèse de valve cardiaque conçue pour une implantation transcathéter, comprenant un bâti de valve (3) repliable sur lequel sont fixés plusieurs feuillets de valve (2), et un anneau de fixation (10) repliable qui se raccorde de manière axiale au bâti de valve (3) et est conçu pour une mise en place et une fixation par serrage dans un vaisseau sanguin, **caractérisée en ce que** l'anneau de fixation (10) est relié au bâti de valve (3) avec désaccouplement de force de telle manière que, lors de la fixation dans un vaisseau sanguin, une déformation de l'anneau de fixation (10) ne soit transmise pas plus que de manière limitée au bâti de valve (3), et le bâti de valve (3) définit plusieurs commissures (4), avec des coudes de liaison (6) situés entre lesdites commissures et auxquels sont fixés les feuillets de valve (2), les régions sommitales des coudes de liaison (6) faisant saillie de manière radiale par rapport à l'anneau de fixation (10) dans l'état déplié de la prothèse de valve cardiaque, de sorte que, dans la région centrale de chaque coude (6), qui définit la région sommitale, le coude (6) forme une protubérance orientée radialement vers l'extérieur par rapport à l'anneau de fixation et à une extrémité libre du bâti de valve (3) qui, à l'état implanté, peuvent s'étendre dans les sinus aortiques.

2. Prothèse de valve cardiaque selon la revendication 1, **caractérisée en ce que** l'anneau de fixation (10) est situé en amont du bâti de valve (3) dans le sens d'écoulement prévu de la prothèse de valve cardiaque.

3. Prothèse de valve cardiaque selon la revendication 1 ou 2, **caractérisée en ce que** le bâti de valve (3) et/ou l'anneau de fixation (10) sont conçus pour se déployer de manière automatique ou pour se déployer à l'aide d'au moins un ballonnet.

4. Prothèse de valve cardiaque selon l'une quelconque des revendications 1 à 3, **caractérisée en ce que** le bâti de valve (3) et/ou l'anneau de fixation (10) sont réalisés à partir de métal et de manière préférée d'un matériau à mémoire de forme.

5. Prothèse de valve cardiaque selon l'une quelconque des revendications précédentes, **caractérisée en ce que** le bâti de valve (3) et/ou l'anneau de fixation (10) sont réalisés à partir d'un alliage de nickel et de titane, en particulier de nitinol, ou d'un alliage de cobalt et de chrome.

6. Prothèse de valve cardiaque selon l'une quelconque des revendications précédentes, **caractérisée en ce que** le bâti de valve (3) et l'anneau de fixation (10) sont reliés l'un à l'autre par une région de liaison (12) déformable.

7. Prothèse de valve cardiaque selon la revendication 6, **caractérisée en ce que** la région de liaison (12) est réalisée dans le même matériau que le bâti de valve (3) et/ou que l'anneau de fixation (10).

8. Prothèse de valve cardiaque selon la revendication 6 ou 7, **caractérisée en ce que** le bâti de valve (3), la région de liaison (12) et l'anneau de fixation (10) sont réalisés d'un seul tenant.

9. Prothèse de valve cardiaque selon l'une quelconque des revendications 1 à 7, **caractérisée en ce que** le bâti de valve (3), la région de liaison (12) et l'anneau de fixation (10) sont réalisés en plusieurs parties.

10. Prothèse de valve cardiaque selon l'une quelconque des revendications 6 à 9, **caractérisée en ce que** la région de liaison (12) présente une structure ayant une résistance inférieure à celle du bâti de valve (3).

11. Prothèse de valve cardiaque selon la revendication 10, **caractérisée en ce que** la région de liaison (12) présente une structure ayant une épaisseur de matériau plus faible et/ou étant d'une réalisation plus mince et/ou ayant une forme différente de ses éléments de support (16) par rapport à une structure du bâti de valve (3).

12. Prothèse de valve cardiaque selon l'une quelconque des revendications 6 à 11, **caractérisée en ce que** la région de liaison (12) et le bâti de valve (3) sont réalisés respectivement sous la forme d'une structure de grille repliable.

13. Prothèse de valve cardiaque selon la revendication 12, **caractérisée en ce qu'**au moins une partie des fils de grille (16) de la région de liaison (12) sont plus étroits que les fils de grille (18) du bâti de valve (3) et/ou au moins une partie des espaces libres (20) situés entre les fils de grille (16) au sein de la région de liaison (12) sont plus grands que les espaces libres (20) situés entre les fils de grille (18) du bâti de valve (3).

14. Prothèse de valve cardiaque selon l'une quelconque des revendications précédentes, **caractérisée en ce que** le bâti de valve (3) définit plusieurs commissures (4) reliées entre elles à leurs extrémités libres par un anneau de liaison (22) pliable.

15. Prothèse de valve cardiaque selon l'une quelconque des revendications précédentes, **caractérisée en ce que** le bâti de valve (3) et l'anneau de fixation (10) ainsi que la région de liaison (12) les reliant sont gainés et en particulier sont gainés avec un matériau textile.

16. Prothèse de valve cardiaque selon l'une quelconque des revendications précédentes, **caractérisée en ce que** les feuillets de valve (2) sont constitués d'un matériau biologique.

17. Prothèse de valve cardiaque selon l'une quelconque des revendications précédentes, **caractérisée en ce qu'**un ou plusieurs bourrelets de fixation annulaires (24) est/sont réalisé(s) dans la région de l'anneau de fixation (10).
